(19) Europäisches Patentamt / European Patent Office / Office européen des brevets

(11) **EP 2 855 711 B1**

(12) **FASCICULE DE BREVET EUROPEEN**

(45) Date de publication et mention de la délivrance du brevet:
**12.07.2017   Bulletin 2017/28**

(21) Numéro de dépôt: **13728480.8**

(22) Date de dépôt: **24.05.2013**

(51) Int Cl.:
*C12R 1/865* (2006.01)     *C12N 1/16* (2006.01)
*C12N 1/36* (2006.01)     *C12P 7/10* (2006.01)
*C12P 7/06* (2006.01)

(86) Numéro de dépôt international:
**PCT/FR2013/051137**

(87) Numéro de publication internationale:
**WO 2013/178915 (05.12.2013 Gazette 2013/49)**

(54) **SOUCHES DE LEVURE APTES A METABOLISER LE XYLOSE ET RESISTANTES AUX INHIBITEURS, PROCEDE D'OBTENTION ET UTILISATION**

HEFESTÄMME ZUR VERSTOFFWECHSLUNG VON XYLOSE UND MIT RESISTENZ GEGENÜBER INHIBITOREN, VERFAHREN ZUR HERSTELLUNG DAVON UND VERWENDUNG DAVON

YEAST STRAINS CAPABLE OF METABOLIZING XYLOSE AND RESISTANT TO INHIBITORS, METHOD FOR OBTAINING SAME AND USE THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.06.2012   FR 1255076**

(43) Date de publication de la demande:
**08.04.2015   Bulletin 2015/15**

(73) Titulaire: **Lesaffre et Compagnie**
**75001 Paris (FR)**

(72) Inventeurs:
• **DESFOUGERES, Thomas**
  **F-59960 Neuville en Ferrain (FR)**
• **PIGNEDE, Georges**
  **F-59700 Marcq-en-Baroeul (FR)**

(74) Mandataire: **Cabinet Plasseraud**
**66 rue de la Chaussée d'Antin**
**75440 Paris Cedex 09 (FR)**

(56) Documents cités:
**WO-A1-2011/128552     WO-A1-2012/072793**

• **TOMOYA SANDA ET AL: "Repeated-batch fermentation of lignocellulosic hydrolysate to ethanol using a hybridstrain metabolically engineered for tolerance to acetic and formic acids", BIORESOURCE TECHNOLOGY, ELSEVIER BV, GB, vol. 102, no. 17, 6 juin 2011 (2011-06-06) , pages 7917-7924, XP028247765, ISSN: 0960-8524, DOI: 10.1016/J.BIORTECH.2011.06.028 [extrait le 2011-06-12]**

EP 2 855 711 B1

**Description**

DOMAINE TECHNIQUE

La présente invention concerne le domaine des souches de levure aptes à métaboliser le xylose et présentant également une résistance à au moins un inhibiteur de fermentation, leur procédé d'obtention, et l'utilisation de ces souches de levure pour la production d'un produit de fermentation dans un milieu comprenant du xylose, en présence ou non d'au moins un inhibiteur de fermentation.

ARRIERE-PLAN TECHNOLOGIQUE

Le document PCT/EP2011/071616 décrit des levures pour la production d'alcool dans des milieux contenant au moins un pentose, par l'expression de la voie métabolique XI-XDH. Ce document décrit pour la première fois l'association d'une surexpression de l'activité xylose isomérase (XI) avec une surexpression de l'activité xylitol désydrogénase (XDH) et montre que la surexpression de l'activité xylitol désydrogénase permet d'éviter l'inhibition par le xylitol de l'activité xylose isomérase. Le document PCT/EP2011/071616 décrit par exemple la souche de levure déposée à la CNCM le 5 octobre 2011 sous le numéro 1-4538.

La production industrielle d'alcool par la levure à partir d'hydrolysats lignocellulosiques nécessite non seulement la machinerie génétique pour métaboliser les pentoses, mais également des propriétés de résistance aux inhibiteurs de fermentation présents dans ces hydrolysats lignocellulosiques.

Les inhibiteurs de fermentation sont produits pendant le prétraitement et l'hydrolyse de la biomasse lignocellulosique. Parmi les inhibiteurs de fermentation figurent des furaldéhydes (furfural, HMF), des composés phénoliques et des acides organiques (acide acétique, acide lévulinique, acide formique).

Différents moyens ont été décrits pour contrer l'effet des inhibiteurs de fermentation, parmi lesquels la détoxification du milieu de fermentation, l'amélioration du procédé de fermentation, ou l'amélioration de la résistance des levures aux inhibiteurs de fermentation. La résistance des levures aux inhibiteurs de fermentation a par exemple été améliorée génétiquement, par évolution dirigée ou par acclimatation (Almeida et Hahn-Hägerdal, International Sugar Journal, 2009, Vol. 111, n°1323).

Toyoma et al. (Bioresour. Technol., 2011, 12: 7917-7924) décrit des souches de levures fermentant le xylose et résistantes à des acides faibles, tels que l'acide acétique ou l'acide formique, ces souches étant obtenues en augmentant les activités transaldolase (TAL) et formate déshydrogénase (FDH).

Toutefois, il semble difficile de fournir une souche de levure industrielle qui donne des levures à la fois performantes pour la production d'alcool dans un milieu de fermentation comprenant au moins un pentose et résistantes à au moins un inhibiteur de fermentation.

Il existe ainsi un réel besoin de fournir de nouvelles souches de levure qui sont à aptes à métaboliser au moins un pentose, par exemple le xylose, même en présence d'au moins un inhibiteur de la fermentation, tel que l'acide acétique, lesdites souches de levure donnant des levures performantes pour la production d'alcool, même en présence d'au moins un inhibiteur de fermentation.

RESUME DE L'INVENTION

Un objet de l'invention concerne la souche de levure déposée le 24 mai 2012 à la CNCM sous le numéro 1-4627.

Un autre objet de l'invention concerne un procédé d'obtention d'une souche de levure apte à métaboliser le xylose et résistante à l'acide acétique, comprenant les étapes de :

- croisement de la souche de levure déposée le 5 octobre 2011 à la CNCM sous le numéro 1-4538 avec la souche de levure déposée le 24 mai 2012 à la CNCM sous le numéro 1-4627, pour obtenir au moins un hybride,
- sélection d'au moins un hybride apte à métaboliser le xylose et résistant à l'acide acétique.

Le présent document décrit aussi une souche de levure apte à métaboliser le xylose et résistante à l'acide acétique, susceptible d'être obtenue par le procédé tel que défini ci-dessus. Est également décrite ici une souche de levure dérivée d'une souche de levure telle que définie ci-dessus, caractérisée en ce que ladite souche de levure dérivée est apte à métaboliser le xylose et résistante à l'acide acétique.

Est aussi décrite ici une levure obtenue par culture d'une souche de levure telle que définie ci-dessus ou par culture d'une souche de levure dérivée telle que définie ci-dessus.

Un autre objet de l'invention concerne un procédé de production d'au moins un produit de fermentation comprenant une étape de fermentation, en condition anaérobie, par une levure telle que définie ci-dessus.

Un autre objet de l'invention concerne l'utilisation d'une levure telle que définie ci-dessus pour la production d'au moins un produit de fermentation, de préférence dans un milieu de fermentation comprenant du xylose et/ou au moins un inhibiteur de fermentation.

D'autres objets de l'invention concerne une culture comprenant une souche de levure telle que définie ci-dessus, et un pain de levure comprenant une souche de levure telle que définie ci-dessus.

BREVE DESCRIPTION DES FIGURES

Figure 1 : Production d'éthanol (en g / kg de milieu de fermentation) au cours du temps (en heures) dans le milieu de fermentation YFGX-Ac, à 32°C, de la souche de levure 1-4538 (losange), la souche de levure H1 (carré), la souche de levure ED1 (triangle), la souche de levure ED2 (cercle) et la souche de levure ED3 (croix).

Figure 2 : Production d'éthanol (en g / kg de milieu de fermentation) au cours du temps (en heures) dans le milieu A, à 32°C, par la souche de levure 1-4538 (losange), la souche de levure ED1 (triangle) et la souche de levure ED2 (cercle).

Figure 3 : Production d'éthanol (en g / kg de milieu de fermentation) au cours du temps (en heures) dans le milieu A, à 35°C, par la souche de levure 1-4538 (losange), la souche de levure ED1 (triangle), la souche de levure ED2 (cercle) et la souche de levure ED3 (croix).

Figure 4 : Production d'éthanol (en g / kg de milieu de fermentation) au cours du temps (en heures) dans le milieu B, à 35°C, par la souche de levure 1-4538 (losange), la souche de levure ED1 (triangle), la souche de levure ED2 (cercle) et la souche de levure ED3 (croix).

DEFINITIONS

L'expression « souche de levure » désigne une population relativement homogène de cellules de levure.

Une souche de levure est obtenue à partir de l'isolement d'un clone, un clone étant une population de cellules obtenue à partir d'une seule cellule de levure.

Par gène « exogène », on entend un gène qui n'est pas présent naturellement dans une souche de levure de l'espèce considérée.

Par gène « endogène », on entend un gène qui est présent naturellement dans une souche de levure de l'espèce considérée.

Une xylose isomérase ou XI désigne ici une enzyme apte à transformer en une seule étape le D-xylose en D-xylulose et qui correspond à la classe EC 5.3.1.5.

Une xylitol déshydrogénase ou XDH désigne ici une enzyme apte à transformer en une seule étape le xylitol en D-xylulose et qui correspond à la classe EC 1.1.1.9.

La xylose réductase ou XR désigne ici une enzyme apte à transformer en une seule étape le D-xylose en xylitol et qui correspond à la classe EC 1.1.1.307.

Une D-xylulokinase ou XKS désigne ici une enzyme apte à transformer en une seule étape le D-xylulose en D-xylulose-5-phosphate et qui correspond à la classe EC 2.7.1.17.

Le gène GRE3 code pour une enzyme aldose réductase qui correspond à la classe EC 1.1.1.21.

Le gène RPE1 code pour une enzyme D-ribulose-5-phosphate 3-épimerase qui correspond à la classe EC 5.1.3.1.

Le gène RKI1 code pour une enzyme ribose-5-phosphate kétol-isomérase qui correspond à la classe EC 5.3.1.6.

Le gène TKL1 code pour une enzyme transkétolase qui correspond à la classe EC 2.2.1.1.

Le gène TAL1 code pour une enzyme transaldolase qui correspond à la classe EC 2.2.1.2.

Une souche de levure prototrophe est une souche de levure capable de croître sur un milieu minimum. En particulier, une souche de levure prototrophe est capable de synthétiser tous les acides aminés et bases nécessaires à sa croissance.

Un milieu minimum est un milieu comprenant une source de carbone ($C_xH_yO_z$), une source d'azote minéral, une source de potassium, une source de phosphore, une source de soufre, une source de magnésium, une source de calcium, une source de fer, une source d'oligo-éléments et de l'eau.

Un exemple de milieu minimum est le milieu YNB (*Yeast Nitrogen Base*) auquel est ajouté une source de carbone (par exemple un sucre) et une source d'azote minéral (par exemple le sulfate d'ammonium).

Le milieu YNB comprend par litre : biotine 2 μg, pantothénate de calcium 400 μg, acide folique 2 μg, inositol 2000 μg, niacine 400 μg, acide p-aminobenzoïque 200 μg, hydrochlorure de pyridoxine 400 μg, riboflavine 200 μg, hydrochlorure de thiamine 400 μg, acide borique 500 μg, sulfate de cuivre 40 μg, iodure de potassium 100 μg, chlorure ferrique 200 μg, sulfate de manganèse 400 μg, molybdate de sodium 200 μg, sulfate de zinc 400 μg, phosphate de potassium monobasique 1 g, sulfate de magnésium 500 mg, chlorure de sodium 100 mg, chlorure de calcium 100 mg, sulfate d'ammonium 5g/l, pH final 5,4.

Par l'expression « souche de levure dérivée », on désigne une souche de levure dérivée par un ou plusieurs croisements et/ou par mutation et/ou par transformation génétique.

Une souche de levure dérivée par croisement peut être obtenue par croisement d'une souche de levure selon l'invention avec la même souche de levure, avec une autre souche de levure selon l'invention, ou avec une autre souche de levure quelconque.

Une souche de levure dérivée par mutation peut être une souche de levure ayant subi au moins une mutation spontanée dans son génome ou au moins une mutation induite par mutagenèse. La ou les mutations d'une souche dérivée peuvent être silencieuses ou non.

Par l'expression « mutagenèse », on désigne à la fois la mutagenèse aléatoire obtenue par l'application d'un rayonnement (par exemple UV) ou par des agents chimiques mutagènes, et la mutagenèse insertionnelle ou dirigée, par transposition ou par intégration d'un fragment d'ADN exogène.

Une souche de levure dérivée par transformation génétique est une souche de levure dans laquelle a été introduite une séquence d'ADN qui est de préférence apportée par un plasmide ou intégrée directement dans le génome.

DESCRIPTION DETAILLEE DE L'INVENTION

La présente invention a pour objet de fournir une souche de levure apte à métaboliser au moins un pentose, en particulier le xylose, même en présence d'au moins un inhibiteur de fermentation, tel que l'acide acétique.

Une souche de levure apte à métaboliser le xylose au sens de l'invention est une souche de levure apte à produire de l'éthanol dans un milieu comprenant du xylose, en condition anaérobie.

En particulier, une souche de levure apte à métaboliser le xylose est une souche de levure capable de convertir le xylose en éthanol, c'est-à-dire capable de fermenter le xylose.

La conversion du xylose en éthanol résulte de l'isomérisation directe ou indirecte du xylose en xylulose, suivie de l'utilisation du xylulose ainsi obtenu dans la partie non oxydative de la voie des pentoses phosphate.

Une souche de levure apte à métaboliser le xylose au sens de l'invention est une souche de levure qui convertit au moins 70%, de préférence au moins 80%, plus préférentiellement au moins 90 % du xylose en éthanol en 60 heures dans un milieu de fermentation comprenant 55 g de glucose et 45 g de xylose par kg de milieu de fermentation, en condition anaérobie. L'ensemencement de la souche de levure utilisé pour mesurer le pourcentage de xylose converti en éthanol est de préférence 0,25 g en matière sèche / kg de milieu de fermentation. La durée de 60 heures est calculée à partir de l'ensemencement de la souche de levure dans le milieu de fermentation.

Le milieu de fermentation utilisé pour mesurer le pourcentage de xylose converti en éthanol est de préférence un milieu synthétique.

Un milieu synthétique est un milieu dont la composition chimique exacte est connue.

Un milieu synthétique utilisé ici comprend une source de carbone, une source d'azote, une source de phosphore, ainsi que les vitamines et minéraux essentiels à la croissance d'une souche de levure.

Le milieu de fermentation utilisé pour mesurer le pourcentage de xylose converti en éthanol est préférablement le milieu synthétique YFGX comprenant 55 g/kg de glucose, 45 g/kg de xylose, 10 g/kg d'extrait de levure, 10g/kg de peptone, 2000 ppm d'acide acétique, pH ajusté à 5 par KOH.

Dans le milieu YFGX, l'acide acétique présent à une concentration de 2000 ppm à pH 5 n'a pas d'effet inhibiteur.

La fermentation est conduite de préférence à 32°C, sous agitation modérée, par exemple 90 rpm.

L'agitation est modérée pour ne pas être oxygénante.

Le pH du milieu est de préférence contrôlé, par exemple par le pouvoir tampon d'un couple acide/base, par exemple le pouvoir tampon acide acétique/acétate dans le milieu YFGX.

La quantité d'éthanol présente dans le milieu de fermentation est mesurée par tout moyen approprié connu de l'homme du métier.

Il peut s'agir d'une mesure directe de l'éthanol produit ou d'une mesure indirecte via un paramètre corrélé à la production d'éthanol, tel que la perte de masse.

Par exemple, la production d'alcool peut être mesurée par chromatographie, notamment par HPLC (High Performance Liquid Chromatography), un kit enzymatique (par exemple le kit de dosage de l'éthanol de Boehringer), ou un dosage par le dichromate de potassium.

La quantité de xylose présente dans le milieu de fermentation est mesurée par tout moyen approprié connu de l'homme du métier, de préférence par chromatographie, notamment par HPLC.

L'utilisation d'un milieu de fermentation comprenant à la fois du glucose et du xylose permet d'évaluer la conversion du xylose en éthanol à partir d'une quantité de biomasse comparable pour les différentes souches de levure évaluées. En effet, les souches de levure fermentent en premier le glucose du mélange glucose et xylose, puis le glucose et le xylose.

La capacité à métaboliser le xylose en présence d'au moins un inhibiteur de fermentation est qualifiée de résistance audit inhibiteur de fermentation.

Dans le cadre de la présente invention, l'inhibiteur de fermentation est de préférence l'acide acétique.

C'est la forme non ionisée de l'acide acétique, à partir d'une certaine concentration dite concentration toxique, qui est responsable de sa toxicité et donc de son effet inhibiteur.

Par exemple, une concentration de 4000 ppm d'acide acétique à pH 4,4 est une concentration toxique pour des souches non résistantes à l'acide acétique, telles que la souche de levure 1-4538.

Lors d'une fermentation en condition anaérobie, l'effet inhibiteur de l'acide acétique se traduit notamment par un retard de l'initiation de la conversion des sucres en biomasse et en éthanol.

La résistance à l'acide acétique est mesurée ici par rapport au retard d'initiation de la conversion des sucres en éthanol, c'est-à-dire au retard d'initiation de la fermentation alcoolique.

La courbe de fermentation alcoolique représentant la quantité d'alcool produit en fonction du temps comporte généralement trois phases :

- une phase de latence, au cours de laquelle il n'y a pas de production d'éthanol,
- une phase de production d'alcool, et
- une phase de plateau, qui correspond à la fin de la fermentation.

Le retard de l'initiation de la fermentation alcoolique correspond ici à l'abscisse à l'origine de la droite représentant la dérivée maximale de la vitesse de production d'alcool.

Plus simplement, le retard de l'initiation de la fermentation alcoolique correspond ici à l'abscisse à l'origine de la droite correspondant à la pente de la phase de production d'alcool.

Une souche de levure résistante à l'acide acétique est définie ici comme une souche de levure ayant un retard d'initiation de la fermentation alcoolique inférieur à 30 heures, de préférence inférieur à 29 heures, plus préférentiellement inférieur à 28 heures, dans un milieu de fermentation comprenant 4000 ppm d'acide acétique à pH 4,4.

Le milieu de fermentation utilisé pour évaluer la résistance à l'acide acétique est de préférence un milieu synthétique, plus préférentiellement le milieu YFAc.

La composition du milieu YFAc est la suivante : 150 g / kg de glucose, 5 g / kg d'extrait de levure, 4,7 g / kg de DAP (diammonium phosphate), 11,5 g / kg d'acide citrique, 4 g / kg d'acide acétique, 13,5 g / kg de citrate de sodium, 1 ml / kg de Tween 80, 2 ml / kg de $ZnSO_4$ (à 10,6 g/L), 2,5 ml / kg de $MgSO_4$ $7H_2O$ (à 400 g/L), 1 ml / kg de thiamine (à 18,24 g/L), 1 ml / kg de pyridoxine (à 5,28g/L), 1 ml / kg de biotine (à 1,76g/L), 1 ml / kg de panthoténate (à 3,8 g/L), 2,5 ml / kg d'acide nicotinique (à 8 g/L), 1 ml / kg de mésoinositol (à 50 g/L), 1 ml / kg de riboflavine (à 1 g/L), 1 ml / kg de para-aminobenzoate (à 1,2 g/L), pH ajusté à 4,4 avec KOH.

L'ensemencement de la souche de levure utilisé pour évaluer la résistance à l'acide acétique est, de préférence, 0,25 g en matière sèche / kg de milieu de fermentation.

Le temps t=0 de la courbe de fermentation alcoolique correspond au moment de l'ensemencement de la souche de levure dans le milieu de fermentation.

La fermentation alcoolique est conduite en condition anaérobie, de préférence à 32°C et sous agitation modérée, par exemple 90 rpm.

L'agitation est modérée pour ne pas être oxygénante.

Le pH du milieu est de préférence contrôlé, par exemple par le pouvoir tampon d'un couple acide/base, par exemple le pouvoir tampon acide acétique/acétate dans le milieu YFAc.

Dans le milieu de fermentation YFAc, une souche de levure non résistante à l'acide acétique peut avoir un retard d'initiation de la fermentation alcoolique d'au moins 40h.

Les Inventeurs ont cherché à obtenir une souche de levure apte à métaboliser le xylose, même en présence d'acide acétique, en partant d'une souche de levure performante en terme de conversion du xylose en éthanol.

Cette souche de levure performante en terme de conversion du xylose en éthanol choisie comme souche de départ à améliorer en vue de lui conférer un caractère de résistance à l'acide acétique est la souche de levure 1-4538 déposée en vertu du traité de Budapest le 5 octobre 2011 auprès de la CNCM (*Collection Nationale de Cultures de Microorganismes*), 25, rue du Docteur Roux, 75724 Paris cedex 15, France.

La souche de levure 1-4538 convertit au moins 90 % du xylose en éthanol en 60 heures dans le milieu de fermentation YFGX comprenant 55 g de glucose et 45 g de xylose par kg de milieu.

Cependant, la souche de levure 1-4538 est particulièrement sensible à l'acide acétique : elle a un retard d'initiation de la fermentation alcoolique d'au moins 40 heures dans un milieu de fermentation YFAc comprenant 4000 ppm d'acide acétique à pH 4,4.

La souche de levure 1-4538 est une souche de *Saccharomyces cerevisiae* obtenue par évolution dirigée à partir d'une souche de levure génétiquement modifiée par :

- l'insertion d'au moins une copie d'un gène exogène codant pour une xylose isomérase (XI) de *Clostridium phyto-*

*fermentans* sous la dépendance du promoteur pADH1 et du terminateur CYC1,

- l'insertion d'au moins une copie d'un gène exogène codant pour une xylitol déshydrogénase (XDH) de *Pichia stipitis,* sous la dépendance du promoteur pADH1 et du terminateur CYC1,
- au moins une copie du gène endogène TAL1 placé sous la dépendance du promoteur pPGK1,
- au moins une copie du gène endogène TKL1 placé sous la dépendance du promoteur pTDH3,
- au moins une copie du gène endogène RPE1 placé sous la dépendance du promoteur pTDH3,
- au moins une copie du gène endogène RKI1 placé sous la dépendance du promoteur pTDH3,
- l'insertion d'au moins une copie d'un gène endogène codant pour une xylulokinase (XKS1) sous la dépendance du promoteur pADH1 et du terminateur CYC1, et
- la délétion d'au moins une copie du cadre ouvert de lecture du gène endogène GRE3 codant pour une aldose réductase.

La souche de levure 1-4538 est dépourvue de tout marqueur de sélection résiduel.

La souche de levure 1-4538 ne comprend pas de gène codant pour une XR d'origine exogène, ni de gène araA, araB ou AraD.

Le gène exogène codant pour une xylose isomérase (XI) de *Clostridium phytofermentans* est le gène de séquence optimisée SEQ ID NO: 3 décrite dans le document DE102008031350. Le gène exogène codant pour une xylitol déshydrogénase (XDH) de *Pichia stipitis* est le gène de séquence de référence X55392.1 sur Genbank.

Les promoteurs pADH1, pPGK1 et pTDH3 sont des promoteurs de *Saccharomyces cerevisiae.*

Le terminateur CYC1 est un promoteur de *Saccharomyces cerevisiae.*

La souche de levure 1-4538 est une souche de levure industrielle qui est aneuploïde.

La souche de levure 1-4538 est prototrophe.

Afin de conférer une propriété de résistance à l'acide acétique à la souche de levure 1-4538, mais sans modifier sensiblement sa capacité à métaboliser le xylose, les Inventeurs ont choisi d'utiliser la technique de croisement de souches.

A la connaissance des Inventeurs, c'est la première fois que la technique de croisement est utilisée pour améliorer une souche de levure aneuploïde génétiquement modifiée.

En effet, l'homme du métier n'envisagerait pas de prime abord d'utiliser la technique de croisement, alors que la souche de levure de départ :

- comporte pas moins de huit modifications génétiques devant *a priori* être transférées aux ségrégeants sélectionnés,
- est une souche de levure aneuploïde et qu'il existe des problèmes de ségrégation en l'absence de diploïdie stricte, et
- a subi une étape de mutagenèse dans le cadre d'une évolution dirigée, ce qui peut être à l'origine de difficultés pour les croisements, en particulier en cas de translocation chromosomique à l'origine de problèmes de germination.

Des croisements ont ainsi été réalisés entre la souche de levure 1-4538 et des souches de levure résistantes à l'acide acétique et non génétiquement modifiées, c'est-à-dire ne possédant pas la machinerie génétique nécessaire à la fermentation du xylose.

Les souches de levure résistantes à l'acide acétique utilisées pour les croisements ont un retard d'initiation de la fermentation alcoolique inférieur à 14 h dans le milieu de fermentation YFAc.

Les résultats des croisements n'ont pas été concluants : si le caractère de résistance à l'acide acétique était bien transféré aux hybrides, ceux-ci étaient nettement moins performants que la souche de levure 1-4538 de départ pour convertir le xylose en éthanol.

Au mieux, les hybrides obtenus convertissaient 40% du xylose en éthanol en 60 heures dans le milieu de fermentation YFGX comprenant 55 g de glucose et 45 g de xylose par kg de milieu.

Les Inventeurs ont essayé d'améliorer la fermentation du xylose de ces hybrides par évolution dirigée, mais sans davantage de succès.

Les Inventeurs ont alors mis en évidence que, pour transférer le caractère de résistance à l'acide acétique par croisement, sans perdre la capacité à métaboliser efficacement le xylose, il était nécessaire de croiser la souche de levure 1-4538 avec une souche de levure résistante à l'acide acétique qui a un fond génétique proche, c'est-à-dire qui possède également la machinerie génétique pour métaboliser le xylose, mais sans être nécessairement performante pour métaboliser le xylose.

La souche de levure utilisée, qui est à la fois résistante à l'acide acétique et qui possède également la machinerie génétique pour métaboliser le xylose, est la souche de *Saccharomyces cerevisiae* déposée sous le numéro 1-4627 en vertu du traité de Budapest le 24 mai 2012 auprès de la CNCM (*Collection Nationale de Cultures de Microorganismes*), 25, rue du Docteur Roux, 75724 Paris cedex 15, France.

Ainsi, en croisant la souche de levure 1-4538 avec la souche de levure 1-4627, les Inventeurs ont obtenu des souches de levure à la fois performantes pour métaboliser le xylose et résistantes à l'acide acétique.

La présente invention a ainsi pour objet la souche de levure déposée à la CNCM sous le numéro 1-4627.

La souche 1-4627 constitue l'une des deux souches de départ pour obtenir les souches de levure selon l'invention.

La souche de levure 1-4627 est une nouvelle souche de levure, obtenue par un procédé original de croisement d'une souche de levure aneuploïde résistante à l'acide acétique et non génétiquement modifiée avec une souche de levure aneuploïde génétiquement modifiée.

La souche de levure 1-4627 a ainsi été obtenue de la manière suivante :

- sélection d'une souche de levure résistante à l'acide acétique, c'est-à-dire qui a un retard d'initiation de la fermentation alcoolique inférieur à 14 h dans le milieu de fermentation YFAc,
- sélection d'une souche de levure génétiquement modifiée par :

  ◦ l'insertion d'au moins une copie d'un gène exogène codant pour une xylose isomérase (XI) de *Clostridium phytofermentans* sous la dépendance du promoteur pADH1 et du terminateur CYC1,
  ◦ l'insertion d'au moins une copie d'un gène exogène codant pour une xylitol déshydrogénase (XDH) de *Pichia stipitis,* sous la dépendance du promoteur pADH1 et du terminateur CYC1,
  ◦ au moins une copie du gène endogène TAL1 placé sous la dépendance du promoteur pPGK1,
  ◦ au moins une copie du gène endogène TKL1 placé sous la dépendance du promoteur pTDH3,
  ◦ au moins une copie du gène endogène RPE1 placé sous la dépendance du promoteur pTDH3,
  ◦ au moins une copie du gène endogène RKI1 placé sous la dépendance du promoteur pTDH3,
  ◦ l'insertion d'au moins une copie d'un gène endogène codant une xylulokinase (XKS1) sous la dépendance du promoteur pADH1 et du terminateur CYC1, et
  ◦ la délétion d'au moins une copie du cadre ouvert de lecture du gène endogène GRE3 codant pour une aldose réductase,

- croisement de ladite souche de levure résistante à l'acide acétique avec ladite souche de levure génétiquement modifiée, pour obtenir un hybride,
- évolution dirigée dudit hybride, pour obtenir la souche de levure 1-4627.

La souche de levure 1-4627 comporte au moins une copie d'un gène exogène codant pour une xylose isomérase (XI) de *Clostridium phytofermentans* sous la dépendance du promoteur pADH1 et du terminateur CYC1, au moins une copie d'un gène exogène codant pour une xylitol déshydrogénase (XDH) de *Pichia stipitis,* sous la dépendance du promoteur pADH1 et du terminateur CYC1, au moins une copie du gène endogène TAL1 placé sous la dépendance du promoteur pPGK1, au moins une copie du gène endogène TKL1 placé sous la dépendance du promoteur pTDH3, au moins une copie du gène endogène RPE1 placé sous la dépendance du promoteur pTDH3, au moins une copie du gène endogène RKI1 placé sous la dépendance du promoteur pTDH3, au moins une copie d'un gène endogène codant une xylulokinase (XKS1) sous la dépendance promoteur pADH1 et du terminateur CYC1 et au moins une copie du cadre ouvert de lecture du gène endogène GRE3 codant pour une aldose réductase délétée.

Comme pour la souche de levure 1-4538, le gène exogène codant pour une xylose isomérase (XI) de *Clostridium phytofermentans* est le gène de séquence optimisée SEQ ID NO: 3 décrite dans le document DE102008031350 et le gène exogène codant pour une xylitol déshydrogénase (XDH) de *Pichia stipitis* est le gène de séquence de référence X55392.1 sur Genbank.

La souche de levure 1-4627 est dépourvue de tout marqueur de sélection résiduel.

La souche de levure 1-4627 ne comprend pas de gène codant pour une XR d'origine exogène, ni de gène araA, araB ou AraD.

La souche de levure 1-4627 est une souche de levure industrielle et elle est aneuploïde.

La souche de levure 1-4627 est prototrophe.

La résistance à l'acide acétique de la souche de levure 1-4627 est inférieure à celle de la souche de levure résistante à l'acide acétique de départ utilisée pour le croisement, mais sa résistance à l'acide acétique est largement acceptable pour une application industrielle.

Ainsi, la souche de levure 1-4627 a un retard d'initiation de la fermentation alcoolique inférieur à 20 heures dans le milieu de fermentation YFAc.

S'agissant de la performance sur xylose, la souche de levure 1-4627 convertit environ 60 % du xylose en éthanol, en condition anaérobie, en 60 heures, dans le milieu de fermentation YFGX comprenant 55 g de glucose et 45 g de xylose par kg de milieu.

La souche de levure 1-4627 est donc une souche de levure originale qui possède une résistance à l'acide acétique et qui possède la machinerie génétique pour métaboliser le xylose, mais qui n'est pas apte à métaboliser le xylose au sens de l'invention, c'est-à-dire qui est incompatible pour une utilisation industrielle en vue de la production d'éthanol.

Les Inventeurs ont alors procédé au croisement de la souche de levure 1-4538 avec la souche 1-4627, afin d'obtenir une souche de levure apte à métaboliser le xylose et résistante à l'acide acétique.

La présente invention a ainsi pour objet un procédé d'obtention d'une souche de levure apte à métaboliser le xylose et résistante à l'acide acétique, comprenant les étapes de :

- croisement de la souche de levure déposée à la CNCM sous le numéro 1-4538 avec la souche de levure déposée à la CNCM sous le numéro 1-4627, pour obtenir au moins un hybride,
- sélection d'au moins un hybride apte à métaboliser le xylose et résistant à l'acide acétique, pour obtenir une souche de levure apte à métaboliser le xylose et résistante à l'acide acétique.

Comme défini ci-dessus, la souche de levure apte à métaboliser le xylose et résistante à l'acide acétique selon l'invention a les propriétés suivantes :

- elle convertit au moins 70%, de préférence au moins 80%, plus préférentiellement au moins 90 % du xylose en éthanol en 60 heures dans le milieu de fermentation YFGX comprenant 55 g de glucose et 45 g de xylose par kg de milieu de fermentation, en condition anaérobie, et
- elle a un retard d'initiation de la fermentation alcoolique inférieur à 30 heures, de préférence inférieur à 29 heures, plus préférentiellement inférieur à 28 heures, dans le milieu de fermentation YFAc comprenant 4000 ppm d'acide acétique à pH 4,4.

L'étape de croisement est réalisée selon les techniques classiques, comme celles enseignées dans le chapitre 7 « Sporulation and Hydridization of Yeast » par R.R. Fowell, de l'ouvrage de référence « The Yeasts », Volume 1, édité par A.H. Rose et J. S. Harrison, 1969-Academic Press.

Afin d'améliorer l'efficacité du procédé d'obtention d'une souche de levure selon l'invention, il est particulièrement avantageux de réaliser une sélection au niveau des ségrégeants issus de la souche de levure 1-4538 et/ou au niveau des ségrégeants issus de la souche de levure I-4627.

Les ségrégeants de la souche de levure 1-4538 utilisés pour le croisement sont de préférence sélectionnés sur la base de leur capacité à métaboliser le xylose.

Les ségrégeants de la souche de levure 1-4627 utilisés pour le croisement sont sélectionnés sur la base de leur résistance à l'acide acétique.

Le procédé tel que défini ci-dessus, peut être caractérisé en ce que ladite étape de croisement comprend :

- une étape de sporulation de la souche de levure déposée à la CNCM sous le numéro 1-4538, pour obtenir au moins un ségrégeant X,
- une éventuelle étape d'évaluation de la conversion du xylose en éthanol par au moins ségrégeant X,
- une étape de sporulation de la souche de levure déposée à la CNCM sous le numéro 1-4627, pour obtenir au moins un ségrégeant Y,
- une éventuelle étape d'évaluation de la résistance à l'acide acétique d'au moins un ségrégeant Y,
- une étape d'hybridation d'au moins un ségrégeant X avec au moins un ségrégeant Y, ledit ségrégeant X étant capable de convertir du xylose en éthanol et/ ou ledit ségrégeant Y possédant une résistance à l'acide acétique, pour obtenir au moins un hybride.

Les étapes de sporulation, d'évaluation de la conversion du xylose en éthanol et d'évaluation de la résistance à l'acide acétique peuvent être réalisées dans n'importe quel ordre souhaité, à partir du moment où la sporulation d'une souche de levure donnée est effectuée avant l'évaluation de ses ségrégeants.

Par exemple, un procédé tel que défini ci-dessus, peut-être caractérisé en ce que ladite étape de croisement comprend :

- une étape de sporulation de la souche de levure déposée à la CNCM sous le numéro 1-4538, pour obtenir au moins un ségrégeant X,
- une éventuelle étape de mesure du pourcentage de xylose converti en éthanol par au moins un ségrégeant X, en condition anaérobie, en 60 heures dans un milieu de fermentation comprenant 55 g de glucose et 45 g de xylose par kg dudit milieu,
- une étape de sporulation de la souche de levure déposée à la CNCM sous le numéro 1-4627, pour obtenir au moins un ségrégeant Y,
- une éventuelle étape de mesure du retard d'initiation de la fermentation alcoolique d'au moins un ségrégeant Y dans un milieu de fermentation comprenant 4000 ppm d'acide acétique à pH 4,4,

- une étape d'hybridation d'au moins un ségrégeant X avec au moins un ségrégeant Y, le ségrégeant X convertissant au moins 60 % du xylose en éthanol en 60 heures et/ou le ségrégeant Y ayant un retard d'initiation de la fermentation alcoolique inférieur à 30 heures, pour obtenir au moins un hybride.

Les étapes de sporulation, de mesure du pourcentage de xylose converti en éthanol et de mesure du retard d'initiation de la fermentation alcoolique peuvent être réalisées dans n'importe quel ordre souhaité, à partir du moment où la sporulation d'une souche de levure donnée est effectuée avant la mesure du paramètre de sélection appliqué aux ségrégeants de cette souche.

L'ensemencement utilisé pour mesurer le pourcentage de xylose converti en éthanol par au moins un ségrégeant de la souche de levure 1-4538 est de préférence 0,25 g de matière sèche / kg de milieu de fermentation.

Le milieu de fermentation utilisé pour mesurer le pourcentage de xylose converti en éthanol par au moins ségrégeant de la souche de levure 1-4538 est de préférence le milieu de fermentation YFGX.

Les ségrégeants de la souche de levure 1-4538 sont naturellement moins performants que la souche aneuploïde de départ pour métaboliser le xylose. C'est pourquoi le critère de sélection appliqué concernant la conversion du xylose en éthanol est moins exigeant que la capacité de la souche de levure 1-4538 de départ.

Ainsi, un ségrégeant de la souche de levure 1-4538 peut être sélectionné s'il convertit, en condition anaérobie, au moins 60% du xylose en éthanol en 60 heures dans un milieu de fermentation comprenant 55 g de glucose et 45 g de xylose par kg dudit milieu, de préférence au moins 65% du xylose, plus préférentiellement encore au moins 70% du xylose.

Afin d'optimiser la durée de l'étape de sélection des ségrégeants, il est possible de mesurer la conversion du xylose en éthanol des ségrégeants de la souche de levure 1-4538 sur une durée plus courte, par exemple sur 48 heures.

Ainsi, un ségrégeant de la souche de levure 1-4538 peut être sélectionné s'il convertit, en condition anaérobie, au moins 60% du xylose en éthanol en 48 heures dans un milieu de fermentation comprenant 55 g de glucose et 45 g de xylose par kg dudit milieu, de préférence au moins 65% du xylose, plus préférentiellement encore au moins 70% du xylose.

Un ségrégeant de la souche de levure 1-4627 peut être sélectionné s'il a un retard d'initiation de la fermentation alcoolique inférieur à 30 heures, de préférence inférieur à 29 heures, plus préférentiellement inférieur à 28 heures, dans un milieu de fermentation comprenant 4000 ppm d'acide acétique à pH 4,4.

Toutefois, de manière surprenante, la majorité des ségrégeants de la souche de levure 1-4627 sont plus résistants à l'acide acétique que la souche de levure de départ 1-4627, c'est-à-dire que leur retard d'initiation de la fermentation alcoolique est inférieur à celui de la souche de levure de départ 1-4627.

Le critère de sélection appliqué concernant la résistance à l'acide acétique est donc de préférence plus exigeant.

Ainsi, un ségrégeant de la souche de levure 1-4627 peut être sélectionné s'il a un retard d'initiation de la fermentation alcoolique inférieur à 25 heures, de préférence inférieur à 20 heures.

Le milieu de fermentation utilisé pour mesurer le retard d'initiation de la fermentation alcoolique d'au moins un ségrégeant de la souche de levure 1-4627 est de préférence le milieu de fermentation YFAc.

L'ensemencement utilisé pour mesurer le retard d'initiation de la fermentation alcoolique d'au moins un ségrégeant de la souche de levure 1-4627 est de préférence 0,25 g de matière sèche / kg de milieu de fermentation.

Les ségrégeants de la souche de levure 1-4627 peuvent en outre être sélectionnés sur leur capacité à convertir le xylose en éthanol.

Toutefois, de manière surprenante, les meilleurs hybrides ont été obtenus à partir de ségrégeants de la souche 1-4627 sélectionnés uniquement sur la base de leur résistance à l'acide acétique.

De préférence, l'étape d'hybridation est réalisée avec :

- au moins un ségrégeant X convertissant au moins 60 % du xylose en éthanol, de préférence au moins 65% du xylose, plus préférentiellement encore au moins 70% du xylose en 60 heures, de préférence en 48 heures, et
- au moins un ségrégeant Y ayant un retard d'initiation de la fermentation alcoolique inférieur à 30 heures, de préférence inférieur à 29 heures, plus préférentiellement inférieur à 28 heures, plus préférentiellement inférieur à 25 heures, plus préférentiellement encore inférieur à 20 heures.

Les hybrides ainsi obtenus sont ensuite sélectionnés sur la base des deux critères : leur capacité à métaboliser le xylose et leur résistance à l'acide acétique.

Un procédé tel que défini ci-dessus, peut être caractérisé en ce que ladite étape de sélection d'au moins un hybride apte à métaboliser le xylose et résistant à l'acide acétique comprend les étapes de :

- mesure du pourcentage de xylose converti en éthanol par au moins un hybride en condition anaérobie en 60 heures dans un milieu de fermentation comprenant 55 g de glucose et 45 g de xylose par kg de milieu de fermentation,
- mesure du retard d'initiation de la fermentation alcoolique d'au moins un hybride dans un milieu de fermentation comprenant 4000 ppm d'acide acétique à pH 4,4,

- sélection d'au moins un hybride qui convertit au moins 70%, de préférence au moins 80%, plus préférentiellement au moins 90 % du xylose en éthanol en 60 heures et dont le retard d'initiation de la fermentation alcoolique est inférieur à 30 heures, de préférence inférieur à 29 heures, plus préférentiellement inférieur à 28 heures, pour obtenir une souche de levure apte à métaboliser le xylose et résistante à l'acide acétique.

L'ordre des étapes de mesure du pourcentage de xylose converti en éthanol et de mesure du retard d'initiation de la fermentation alcoolique n'a pas d'importance : elles peuvent être réalisées l'une après l'autre, dans un sens ou dans l'autre sens, ou en même temps.

Les conditions de mesure du pourcentage de conversion du xylose en éthanol et du retard d'initiation de la fermentation alcoolique sont telles que définies ci-dessus.

Est décrite ici un procédé-d'obtention d'une souche de levure apte à métaboliser le xylose et résistante à l'acide acétique, comprenant les étapes de :

- croisement de la souche de levure déposée à la CNCM sous le numéro 1-4538 avec la souche de levure déposée à la CNCM sous le numéro 1-4627, ladite étape de croisement comprenant les étapes de :

  ○ sporulation de la souche de levure déposée à la CNCM sous le numéro 1-4538, pour obtenir au moins un ségrégeant X,
  ○ mesure du pourcentage de xylose converti en éthanol par au moins un ségrégeant X en 60 heures, de préférence en 48 heures, dans un milieu de fermentation comprenant 55 g de glucose et 45 g de xylose par kg dudit milieu,
  ○ sélection d'au moins un ségrégeant X qui convertit au moins 60 % du xylose en éthanol en 60 heures, de préférence en 48 heures,
  ○ sporulation de la souche de levure déposée à la CNCM sous le numéro 1-4627, pour obtenir au moins un ségrégeant Y,
  ○ mesure du retard d'initiation de la fermentation alcoolique par au moins un ségrégeant Y dans un milieu de fermentation comprenant 4000 ppm d'acide acétique à pH 4,4,
  ○ sélection d'au moins un ségrégeant Y qui a un retard d'initiation de la fermentation alcoolique inférieur à 30 heures,

- hybridation d'au moins un ségrégeant X convertissant au moins 60% du xylose en éthanol en 60 heures, de préférence en 48 heures, avec au moins un ségrégeant Y ayant un retard d'initiation de la fermentation alcoolique inférieur à 30 heures, pour obtenir au moins un hybride,
- sélection d'au moins un hybride apte à métaboliser le xylose et résistant à l'acide acétique, ladite étape de sélection comprenant les étapes de :

  ○ mesure du pourcentage de xylose converti en éthanol par au moins un hybride, en condition anaérobie, en 60 heures, dans un milieu de fermentation comprenant 55 g de glucose et 45 g de xylose par kg de milieu de fermentation,
  ○ mesure du retard d'initiation de la fermentation alcoolique dudit au moins un hybride dans un milieu de fermentation comprenant 4000 ppm d'acide acétique à pH 4,4,
  ○ sélection d'au moins un hybride qui convertit au moins 70%, de préférence au moins 80%, plus préférentiellement au moins 90 % du xylose en éthanol en 60 heures et dont le retard d'initiation de la fermentation alcoolique est inférieur à 30 heures, de préférence inférieur à 29 heures, plus préférentiellement inférieur à 28 heures,

  pour obtenir une souche de levure apte à métaboliser le xylose et résistante à l'acide acétique.

Comme indiqué ci-dessus, l'ordre des étapes de l'étape de croisement n'a pas d'importance, de même que l'ordre des étapes de l'étape de sélection d'au moins un hybride.

De préférence, l'étape d'hybridation est réalisée entre :

- au moins un ségrégeant X convertissant au moins 60% du xylose en éthanol, de préférence au moins 65% du xylose, plus préférentiellement encore au moins 70% du xylose, en 60 heures, de préférence en 48 heures, et
- au moins un ségrégeant Y ayant un retard d'initiation de la fermentation alcoolique inférieur à 30 heures, de préférence inférieur à 29 heures, plus préférentiellement inférieur à 28 heures, plus préférentiellement encore inférieur à 25 heures, plus préférentiellement encore inférieur à 20 heures.

Le procédé peut éventuellement comprendre une étape d'évolution dirigée.

L'étape d'évolution dirigée peut être effectuée sur un hybride non sélectionné lors de l'étape de sélection du procédé d'obtention d'une souche de levure apte à métaboliser le xylose et résistante à l'acide acétique tel que défini ci-dessus, en vue de rendre ledit hybride apte à métaboliser le xylose en éthanol et/ou de le rendre résistant à l'acide acétique.

Est également décrit ici un procédé tel que défini ci-dessus d'obtention d'une souche de levure apte à métaboliser le xylose et résistante à l'acide acétique, comprenant les étapes de :

- croisement de la souche de levure déposée à la CNCM sous le numéro 1-4538 avec la souche de levure déposée à la CNCM sous le numéro 1-4627, pour obtenir au moins un hybride,
- évolution dirigée d'au moins un hybride,
- sélection d'au moins un hybride apte à métaboliser le xylose et résistant à l'acide acétique, pour obtenir une souche de levure apte à métaboliser le xylose et résistante à l'acide acétique.

L'étape d'évolution dirigée comprend les sous-étapes suivantes :

- mutagenèse, pour obtenir des mutants, et
- multiplication des mutants, en cultures cycliques, en condition d'hypoxie, dans un milieu de culture comportant du xylose et/ou un inhibiteur de fermentation.

Pour simplifier, les mutants obtenus à l'issue de l'étape dévolution dirigée sont toujours qualifiés d'hybrides.

L'expression « en condition d'hypoxie » signifie en condition de carence d'oxygène.

Une carence en oxygène est définie ici par un pourcentage en oxygène inférieur à 20% de l'air.

L'étape de mutagenèse est de préférence une mutagenèse dite modérée, c'est-à-dire obtenue par exposition des cellules de levure à un rayonnement UV à 254 nm compris de 100 à 500J/cm$^2$, par exemple 300J/cm$^2$.

Ces conditions n'entrainent qu'un taux de mortalité de 7% à 16% de la population de cellules soumise au rayonnement UV.

Le taux de mortalité est déterminé en étalant sur des boîtes de milieu dont la source de carbone est du glucose, un volume identique de la suspension cellulaire avant et après mutagenèse et en comptant le nombre de colonies après 48 heures de croissance.

La multiplication des mutants en cultures cycliques, en condition d'hypoxie, dans un milieu de culture comprenant du xylose et/ou un inhibiteur comprend, par exemple :

a) la culture de la population cellulaire soumise à la mutagenèse, dans un milieu de culture, sous agitation, en condition d'hypoxie, de préférence à 32°C,

b) le prélèvement d'un volume de la culture obtenue à l'étape a), par exemple un ml, et son ré-ensemencement dans un milieu de culture de même composition qu'à l'étape a),

les étapes a) et b) étant répétées au moins 3 fois, de préférence au moins 4 fois, par exemple 6 fois, 7 fois ou 8 fois.

Lorsqu'un inhibiteur de fermentation est présent dans le milieu de culture lors de la multiplication des mutants, il s'agit de préférence de l'acide acétique.

La multiplication des mutants en cultures cycliques en condition d'hypoxie peuvent être réalisées dans un milieu de culture comprenant du xylose et en l'absence d'acide acétique.

Le milieu de culture pour la multiplication des mutants comprend par exemple 7% de xylose. Un exemple de milieu de culture pour la multiplication des mutants est le milieu YFX de composition suivante : 70 g / kg de xylose, 5 g / kg d'extrait de levure, 4,7 g / kg de DAP (diammonium phosphate), 11,5 g / kg d'acide citrique, 13,5 g / kg de citrate de sodium, 1 ml / kg de Tween 80, 2 ml / kg de ZnSO$_4$ (à 10,6 g/L), 2,5 ml / kg de MgSO$_4$ 7H$_2$O (à 400 g/L), 1 ml / kg de thiamine (à 18,24 g/L), 1 ml / kg de pyridoxine (à 5,28g/L), 1 ml / kg de biotine (à 1,76g/L), 1 ml / kg de panthoténate (à 3,8 g/L), 2,5 ml / kg d'acide nicotinique (à 8 g/L), 1 ml / kg de mésoinositol (à 50 g/L), 1 ml / kg de riboflavine (à 1 g/L), 1 ml / kg de para-aminobenzoate (à 1,2 g/L), pH ajusté à 4,4 avec KOH.

Les conditions d'hypoxie sont par exemple obtenues grâce à une surpression partielle dans l'équipement utilisé (par exemple, fioles ou fermenteurs) due à une surpression consécutive à la production de CO$_2$ produit lors de la fermentation, et à l'absence d'aération.

De préférence, la durée de la culture a) est telle que la totalité du xylose du milieu est consommée.

La durée d'une culture est par exemple de 1 semaine à 48 heures.

Le nombre de cultures réalisé est variable, par exemple de 5 à 20.

L'étape d'évolution dirigée peut comprendre une sous-étape de sélection des mutants non-déficients respiratoires.

Un mutant non-déficient respiratoire est un mutant capable de se multiplier, en métabolisme oxydatif.

En particulier, un mutant non-déficient respiratoire est capable de se multiplier, en condition aérobie, à 30°C, dans un milieu qui contient du glycérol comme unique source de carbone, par exemple un milieu comprenant 20g/kg de glycérol, 5g/kg de sulfate d'ammonium et 1,7 g/kg de milieu YNB (*Yeast Nitrogen Base*), par exemple le milieu YNB de Difco.

Lorsque le milieu de culture ne comprend pas d'inhibiteur de fermentation, une sous-étape de multiplication dans un milieu de culture comprenant de l'acide acétique peut être effectuée après certaines ou chaque sous-étape de multiplication en cultures cycliques, afin de vérifier que le milieu contient toujours au moins un mutant résistant à l'acide acétique.

La durée de la multiplication dans un milieu de culture comprenant de l'acide acétique est de préférence supérieure à 20h et inférieure à 75h.

Par exemple, la durée de la sous-étape de multiplication dans un milieu de culture comprenant de l'acide acétique est comprise de 70h à 75h.

Il est également possible de réaliser une étape de sélection des mutants qui ont une vitesse de croissance appropriée, par culture dans un milieu comprenant du glucose, par exemple un milieu comprenant 20g/L de glucose, et sélection des mutants qui ont la vitesse de croissance appropriée.

Une étape d'évolution dirigée peut également être effectuée à partir de l'hybride sélectionné à l'issue de l'étape de sélection du procédé d'obtention d'une souche de levure apte à métaboliser le xylose et résistante à l'acide acétique tel que défini ci-dessus, par exemple afin d'améliorer davantage son aptitude à métaboliser le xylose et/ou sa résistance à l'acide acétique.

Est décrite ici une souche de levure apte à métaboliser le xylose et résistante à l'acide acétique, susceptible d'être obtenue par le procédé tel que défini ci-dessus.

La souche de levure H1 a ainsi été obtenue par le procédé d'obtention d'une souche de levure tel que défini ci-dessus (cf. exemple 1).

La souche de levure H1 convertit au moins 90% du xylose en éthanol en condition anaérobie en 60 heures dans le milieu YFGX et a un retard d'initiation de la fermentation alcoolique inférieur à 22h dans le milieu de fermentation YFAc.

Est également décrite ici une souche de levure apte à métaboliser le xylose et résistante à l'acide acétique, obtenue par le procédé tel que défini ci-dessus, qui est choisie parmi la souche de levure déposée à la CNCM sous le numéro 1-4624, la souche de levure déposée à la CNCM sous le numéro 1-4625 et la souche de levure déposée à la CNCM sous le numéro I-4626.

Les souches I-4624, I-4625 et 1-4626 sont des souches de *Saccharomyces cerevisiae* déposées en vertu du Traité de Budapest le 24 mai 2012 auprès de la CNCM (*Collection Nationale de Cultures de Microorganismes*), 25, rue du Docteur Roux, 75724 Paris cedex 15, France.

La souche de levure numéro 1-4624 convertit au moins 90% du xylose en éthanol en condition anaérobie en 60 heures dans le milieu YFGX et a un retard d'initiation de la fermentation alcoolique inférieur à 23 heures dans le milieu de fermentation YFAc.

La souche de levure numéro 1-4625 convertit au moins 90% du xylose en éthanol en condition anaérobie en 60 heures dans le milieu YFGX et a un retard d'initiation de la fermentation alcoolique inférieur à 23 heures dans le milieu de fermentation YFAc.

La souche de levure numéro 1-4626 convertit au moins 90% du xylose en éthanol en condition anaérobie en 60 heures dans le milieu YFGX et a un retard d'initiation de la fermentation alcoolique inférieur à 27 heures dans le milieu de fermentation YFAc.

Est aussi décrite ici une souche de levure dérivée d'une souche de levure selon l'invention qui partage les mêmes propriétés.

Ainsi, une souche de levure dérivée d'une souche de levure selon l'invention, est caractérisée en ce que ladite souche de levure dérivée est apte à métaboliser le xylose et est résistante à l'acide acétique.

Une souche de levure dérivée est telle que définie ci-dessus dans les définitions.

Est également décrite ici une levure obtenue par culture d'une souche de levure telle que définie ci-dessus ou par culture d'une souche de levure dérivée telle que définie ci-dessus.

Les levures sont obtenues par culture d'une souche de levure selon l'invention ou d'une souche de levure dérivée, notamment comme décrit dans le livre de référence « Yeast Technology », 2ème édition, 1991, G. Reed et T.W. Nagodawithana, publié par Van Nostrand Reinhold, ISBN 0-442-31892-8.

La multiplication des levures, à l'échelle industrielle, comprend en général au moins les deux premières étapes de l'ensemble des étapes suivantes :

- multiplication d'une souche de levure en plusieurs stades, d'abord en semi-anaérobiose, puis en aérobiose,
- séparation par centrifugation de la levure ainsi produite de son milieu de culture, pour obtenir une crème de levure liquide contenant environ entre 12 et 25% de matière sèche, voire une quantité plus élevée de matière sèche si la crème de levure est mélangée avec des produits osmolytes,

- filtration de la crème de levure liquide ainsi obtenue, en général sur un filtre rotatif sous vide, pour obtenir une levure fraîche déshydratée contenant de 26% à 35% de matière sèche,
- malaxage de ladite levure fraîche déshydratée, pour obtenir une masse homogène,
- extrusion de la levure ainsi obtenue, pour obtenir :

   ○ une levure pressée sous forme de pains de levure fraîche ou de levure fraîche émiettée, contenant environ 30% de matière sèche, ou
   ○ une levure sous forme de particules, en général de granules, si la levure est destinée à être séchée,

- éventuellement, séchage de manière ménagée, dans un courant d'air chaud, par exemple par fluidisation, des particules de levures obtenues par extrusion pour obtenir de la levure sèche.

L'étape de séchage est de préférence un séchage rapide ménageant en présence d'un émulsifiant.

Parmi les émulsifiants pouvant être utilisés lors de l'étape de séchage, on peut choisir le monostéarate de sorbitan, utilisé par exemple à une concentration d'environ 1,0% (en poids sur le poids de levure sèche).

Les levures selon l'invention peuvent être utilisées sous toute forme possible.

Par exemple, une levure telle que définie ci-dessus, peut être caractérisée en ce qu'elle est sous forme de crème de levure, de levure pressée, de levure sèche ou de levure surgelée.

Les levures décrites ici ont les mêmes propriétés que la souche de levure à partir de laquelle elles sont obtenues par culture, à savoir :

- les levures décrites ici sont aptes à métaboliser le xylose et
- les levures décrites ici sont résistantes à l'acide acétique.

Les levures décrites ici sont particulièrement intéressantes pour la production d'alcool industriel, par exemple destiné aux biocarburants ou aux industries chimiques.

Est aussi décrit ici un procédé de production d'au moins un produit de fermentation comprenant une étape de fermentation, en condition anaérobie, par une levure telle que définie ci-dessus dans un milieu de fermentation.

Le produit de fermentation est notamment choisi parmi l'éthanol, un métabolite obtenu à partir de l'éthanol ou un métabolite secondaire.

Un produit de fermentation préféré est l'éthanol.

La production d'éthanol est obtenue par fermentation alcoolique.

L'homme du métier sait déterminer les conditions appropriées pour une fermentation alcoolique.

A titre d'exemple, on peut se référer aux conditions de fermentation alcoolique décrites dans le livre de référence « Yeast Technology », 2ème édition, 1991, G. Reed et T.W. Nagodawithana, publié par Van Nostrand Reinhold, ISBN 0-442-31892-8.

Le milieu de fermentation comprend les éléments suivants : au moins une source de carbone fermentescible, au moins une source d'azote, au moins une source de soufre, au moins une source de phosphore, au moins une source de vitamines et/ou au moins une source de minéraux.

La source de carbone est par exemple apportée sous la forme d'un sucre immédiatement assimilable par la levure, un pentose tel que le xylose, du glycérol, de l'éthanol et/ou un mélange de ceux-ci.

La source de carbone est de préférence apportée par un sucre immédiatement assimilable par la levure et par du xylose.

Un sucre immédiatement assimilable par la levure est par exemple un sucre simple de type glucose, fructose ou galactose, un disaccharide de type saccharose et/ou un mélange de ces sucres.

La source de carbone peut être apportée sous la forme d'un sirop de glucose, d'un sirop de fructose, de mélasses, d'EP2 (Egout Pauvre issu de la 2ème cristallisation du sucre), d'un hydrolysat de tout ou partie d'un matériel végétal et/ou d'un mélange de ceux-ci.

La source d'azote est par exemple apportée sous la forme de sulfate d'ammonium, hydroxyde d'ammonium, di-ammonium phosphate, ammoniac, urée et/ou une combinaison de ceux-ci. La source de soufre est par exemple apportée sous la forme de sulfate d'ammonium, sulfate de magnésium, acide sulfurique et/ou une combinaison de ceux-ci.

La source de phosphore est par exemple apportée sous la forme d'acide phosphorique, phosphate de potassium, di-ammonium phosphate, mono-ammonium phosphate, et/ou une combinaison de ceux-ci.

La source de vitamines est par exemple apportée sous la forme de mélasse, hydrolysat de levure, solution de vitamine pure ou d'un mélange de vitamines pures et/ou une combinaison de ceux-ci.

La source de vitamines apporte à la levure l'ensemble des vitamines dans des quantités au moins équivalentes à celles recommandées dans les ouvrages de référence. Plusieurs sources de vitamines peuvent être associées.

La source de minéraux est par exemple apportée sous la forme de mélasse, un mélange de sels minéraux et/ou leur combinaison.

La source de minéraux apporte à la levure l'ensemble des macroéléments et oligoéléments dans des quantités au moins équivalentes à celles recommandées dans les ouvrages de référence. Plusieurs sources de minéraux peuvent être associées.

Une même substance peut apporter plusieurs éléments différents.

Est également décrit ici un procédé tel que défini ci-dessus de production d'au moins un produit de fermentation, de préférence l'éthanol, comprenant une étape de fermentation, en condition anaérobie, par une levure telle que définie ci-dessus dans un milieu de fermentation comprenant du xylose et/ou au moins un inhibiteur de fermentation.

L'inhibiteur de fermentation est par exemple choisi parmi un acide organique, le furfural, le HMF (hydroxy-méthyl-furfural), un ou plusieurs composés phénoliques, la pression osmotique.

L'acide organique est par exemple choisi parmi l'acide acétique, l'acide lactique, l'acide formique, l'acide lévulinique.

Ledit au moins un inhibiteur de fermentation est préférablement l'acide acétique.

La pression osmotique existe par exemple dans un hydrolysat de tout ou partie d'un matériel végétal. La pression osmotique provient alors notamment de la charge saline apportée au cours du procédé d'obtention du dit hydrolysat.

Le milieu de fermentation est tel que défini ci-dessus.

Le milieu de fermentation comprend, de préférence, au moins 0,2% de xylose, le pourcentage étant exprimé en poids / poids du milieu de fermentation.

Un milieu de fermentation préféré comprend 2% à 7% de xylose.

Le milieu de fermentation peut comprendre en outre un sucre immédiatement assimilable par la levure, par exemple du glucose.

Le milieu de fermentation comprend par exemple de 2 à 7% de xylose et de 0,5% à 15% de glucose.

Le milieu de fermentation peut comprendre du xylose, du glucose et de l'acide acétique.

Est également décrit ici un procédé tel que défini ci-dessus de production d'au moins un produit de fermentation, de préférence l'éthanol, caractérisé en ce que ledit milieu de fermentation comprend au moins un hydrolysat de tout ou partie d'un matériel végétal.

Un hydrolysat de tout ou partie d'un matériel végétal peut être obtenu par une étape de prétraitement du matériel végétal, par exemple à température élevée et en présence d'acides ou de solvants organiques, éventuellement suivie d'une hydrolyse partielle ou totale des polymères de sucre, par voie enzymatique et/ou chimique et/ou thermique.

L'hydrolysat de tout ou partie d'un matériel végétal comprend donc un mélange de sucres provenant de l'hydrolyse des polymères de sucres, tels que la cellulose, l'hémicellulose et l'amidon.

Un milieu de fermentation peut également comprendre un hydrolysat de tout ou partie d'un matériel végétal et du saccharose.

Est également décrite ici l'utilisation d'une levure telle que définie ci-dessus pour la production d'au moins un produit de fermentation, de préférence dans un milieu de fermentation comprenant du xylose et/ou au moins un inhibiteur de fermentation.

Le produit de fermentation est tel que défini ci-dessus.

De préférence, le produit de fermentation est l'éthanol.

Le milieu de fermentation est tel que défini ci-dessus.

L'inhibiteur de fermentation est tel que défini ci-dessus.

D'autres caractéristiques et avantages de l'invention apparaîtront encore mieux à la lecture des exemples de réalisation suivants qui illustrent l'invention sans la limiter, et pour la compréhension desquels on se reportera aux dessins annexés.

EXEMPLE 1: OBTENTION D'UNE SOUCHE LEVURE APTE A METABOLISER LE XYLOSE ET RESISTANTE A L'ACIDE ACETIQUE

**Matériel et Méthodes**

(i) Milieux de fermentation

Le milieu de propagation utilisé pour la propagation d'une souche de levure comprend 10g/kg d'extrait de levure, 10g/kg de peptone et 20g/kg de glucose.

Le milieu de culture YFGX décrit ci-dessous est utilisé pour mesurer la conversion du xylose en éthanol.

Le milieu de culture YFAc décrit ci-dessous est utilisé pour mesurer la résistance à l'acide acétique.

Le milieu de culture YFGX-Ac décrit ci-dessous est utilisé pour évaluer les souches de levure dans un milieu de culture qui contient à la fois du xylose, du glucose et un inhibiteur de fermentation, l'acide acétique.

|  | YFGX | YFGX-Ac |
|---|---|---|
| Glucose | 55 g/kg | 55 g/kg |
| Xylose | 45 g/kg | 45 g/kg |
| Extrait de levure | 10 g/kg | 10 g/kg |
| Peptone | 10 g/kg | 10 g/kg |
| Acide Acétique | 2000 ppm | 8000 ppm |
| Ajustement pH avec KOH | pH 5 | pH 5 |

Dans le milieu YFGX, la concentration en acide acétique de 2000 ppm à pH 5 n'a pas d'effet inhibiteur.
Par contre, dans le milieu YFGX-Ac, la concentration en acide acétique de 8000 ppm à pH 5 a un effet inhibiteur.

|  | YFAc |
|---|---|
| Glucose | 150 g / kg |
| Extrait de levure | 5 g / kg |
| DAP (Diammonium phosphate) | 4,7 g / kg |
| Acide citrique | 11,5 g / kg |
| Acide acétique | 4 g / kg |
| Citrate de sodium | 13,5 g / kg |
| Tween 80 | 1 ml / kg |
| ZnSO4 (10,6 g/L) | 2 ml / kg |
| MgSO4 7H2O (400 g/L) | 2,5 ml / kg |
| Thiamine (18,24 g/L) Vit B1 | 1 ml / kg |
| Pyridoxine (5,28g/L) Vit B6 | 1 ml / kg |
| Biotine (1,76g/L) | 1 ml / kg |
| Panthoténate (3,8 g/L) | 1 ml / kg |
| Acide nicotinique (8 g/L) | 2,5 ml / kg |
| Mésoinositol (50 g/L) | 1 ml / kg |
| Riboflavine (1 g/L) | 1 ml / kg |
| Para aminobenzoate (1,2 g/L) | 1 ml / kg |
| Ajustement du pH à 4,4 avec KOH | |

|  | YFX |
|---|---|
| Xylose | 70 g / kg |
| Extrait de levure | 5 g / kg |
| DAP (Diammonium phosphate) | 4,7 g / kg |
| Acide citrique | 11,5 g / kg |
| Citrate de sodium | 13,5 g / kg |
| Tween 80 | 1 ml / kg |

(suite)

|  | YFX |
|---|---|
| ZnSO4 (10,6 g/L) | 2 ml / kg |
| MgSO4 7H2O (400 g/L) | 2,5 ml / kg |
| Thiamine (18,24 g/L) Vit B1 | 1 ml / kg |
| Pyridoxine (5,28g/L) Vit B6 | 1 ml / kg |
| Biotine (1,76g/L) | 1 ml / kg |
| Panthoténate (3,8 g/L) | 1 ml / kg |
| Acide nicotinique (8 g/L) | 2,5 ml / kg |
| Mésoinositol (50 g/L) | 1 ml / kg |
| Riboflavine (1 g/L) | 1 ml / kg |
| Para aminobenzoate (1,2 g/L) | 1 ml / kg |
| Ajustement du pH à 4,4 avec KOH | |

(ii) Propagation de la souche de levure

La propagation de la souche de levure est réalisée en deux temps : une étape de pré-culture suivie d'une étape de culture.

La pré-culture est réalisée en ensemençant une öse de la souche de levure dans 5 ml du milieu de propagation défini ci-dessus.

Après 24h de culture à 30°C dans un milieu aéré par agitation, 2 ml de cette pré-culture sont prélevés et ensemencés dans 50 ml de milieu de propagation.

Cette culture est réalisée pendant 24h à 30°C dans un milieu aéré par agitation.

La mesure de la conversion du xylose en éthanol et de la résistance à l'acide acétique est effectuée sur les cellules de levure issue de cette culture.

(iii) Mesure de la conversion du xylose en éthanol

Pour déterminer la conversion du xylose en éthanol, 0,25 g de matière sèche de la souche de levure propagée comme indiqué ci-dessus sont ensemencés par kg de milieu YFGX.

La fermentation est conduite à 32°C, en condition anaérobie, sous agitation modérée.

Les concentrations en éthanol ainsi qu'en xylose et glucose sont mesurées par HPLC.

La formule suivante est ensuite utilisée pour obtenir le taux de conversion du xylose en éthanol :

$$\text{conversion en \%} = \frac{[xylose]_{initiale} - [xylose]_{finale}}{[xylose]_{initiale}} \times 100$$

dans laquelle $[xylose]_{initiale}$ correspond à la concentration en xylose dans le milieu au moment de l'ensemencement de la souche de levure et $[xylose]_{finale}$ à la concentration en xylose dans le milieu de culture à 60 heures (à partir de l'ensemencement).

(iv) Mesure de la résistance à l'acide acétique

La résistance à l'acide acétique d'une souche de levure est déterminée par le retard d'initiation de la fermentation alcoolique.

0,25 g de matière sèche de la souche de levure propagée comme indiqué ci-dessus sont ensemencés par kg de milieu YFAc.

La fermentation est conduite en condition anaérobie, à 32°C sous agitation modérée.

La production d'éthanol est mesurée de manière indirecte par une mesure de la perte de masse de la fiole de fermentation, cette perte de masse étant directement corrélée à la production d'alcool.

La courbe de fermentation alcoolique indiquant la perte de masse en fonction du temps est effectuée.

Le point d'intersection entre l'axe des abscisses et la dérivée maximale de la perte de masse est déterminé : il correspond au retard d'initiation de la fermentation.

A noter que la dérivée maximale de la perte de masse donne le même résultat que la dérivée maximale de la vitesse de production d'alcool, si la courbe de fermentation alcoolique indique la production d'alcool en fonction du temps au lieu de la perte de masse en fonction du temps. Plus la durée obtenue est restreinte, plus le délai d'initiation de la fermentation est faible et plus la souche de levure est résistante à l'acide acétique.

(v) Croisement

Les souches de départ utilisées pour le croisement sont la souche de levure 1-4538 et la souche de levure 1-4627.

Etape 1 : Croissance de la souche de levure

Une öse de la souche de départ (conservée à -80°C) est ensemencée en surface de la gélose d'une boîte de Pétri de milieu A.

La composition du milieu A est la suivante : 10 g d'extrait de levure, 10 g de peptone, 20 g de glucose, 20 g d'agar, pH 6,2 +/- 0,2, eau qsp 1L.

La boîte de Pétri est ensuite mise en incubation pendant 24 heures à 30°C.

Etape 2 : Sporulation sur du milieu 2

Une öse de la culture précédente est ensemencée en surface de la gélose d'une boîte de Pétri de milieu B.

La composition du milieu B est la suivante : 6,5g d'acétate de sodium, 15g d'agar, pH 6,5-7, eau qsp 1L.

La boîte de Pétri est mise en incubation pendant 96 heures à 25°C. La biomasse obtenue en surface de la boîte est ensuite récoltée dans 500$\mu$L d'eau stérile. A 100$\mu$L de cette suspension sont ajoutés 25$\mu$l de zymolyase. La suspension est mise en incubation pendant 30 minutes à 30°C, avant d'être étalée sur boîte gélosée de milieu 1. De préférence, la dissection des tétrades est effectuée 20 minutes plus tard.

Etape 3 : Dissection des tétrades

Les tétrades sont disséquées au micro-manipulateur SINGER, puis la boîte de pétri est mise en incubation pendant 48 heures à 30°C.

Etape 4 : Conservation des ségrégeants

Les ségrégeants sont ensuite conservés à -80°C dans du milieu 1 comprenant 20% de glycérol.

Etape 5 : Sélection des ségrégeants de la souche de levure 1-4538

Les ségrégeants de la souche de levure 1-4538 sont sélectionnés sur leur capacité à convertir le xylose en éthanol.

La conversion du xylose est mesurée comme en (iii), à partir d'un ségrégeant propagé comme en (ii) pour la souche de levure, à part que la mesure est effectuée à 48 heures au lieu de 60 heures.

Les ségrégeants sélectionnés convertissent au moins 70 % du xylose à 48 heures.

Le type sexuel Mat a ou Mat alpha des ségrégeants sélectionnés est déterminé par PCR.

Etape 6 : Sélection des ségrégeants de la souche 1-4627

Les ségrégeants de la souche de levure 1-4627 sont sélectionnés sur la base de leur résistance à l'acide acétique.

La résistance à l'acide acétique est évaluée comme en (iv), à partir d'un ségrégeant propagé comme en (ii) pour la souche de levure.

Les ségrégeants sélectionnés ont un retard d'initiation de la fermentation inférieur à 25 heures.

Le type sexuel Mat a ou Mat alpha des ségrégeants sélectionnés est déterminé par PCR.

Etape 7 : Croisement et sélection des hybrides

Une öse d'un ségrégeant haploïde Mat alpha est ensemencée en surface de la gélose d'une boîte de Pétri de milieu A. Une öse d'un ségrégeant haploïde Mat a est mélangée au dépôt de la souche Mat alpha.

La composition du milieu A est la suivante : 10 g d'extrait de levure, 10 g de peptone, 20 g de glucose, 20g d'agar, pH 6,2 +/- 0,2, eau qsp 1L.

La boîte de Pétri est ensuite mise en incubation pendant 24 heures à 30°C. Une öse du mélange est ensemencée en surface de la gélose d'une boîte de Pétri de milieu A. Cette étape est réitérée à 5 reprises.

Lors du dernier ensemencement, les cellules sont striées de sorte à obtenir des colonies isolées. Sur chaque colonie isolée, des cellules de levure sont prélevée et cultivées, leur ADN génomique est extrait et une PCR est réalisée sur cet ADN génomique, afin de vérifier que les allèles Mat a et Mat alpha sont bien présents dans une même cellule de levure.

Les cellules de levure qui ont les allèles Mat a et Mat alpha sont appelés hybrides.

Les hybrides sont ensuite sélectionnés sur la base de deux critères : leur capacité à métaboliser le xylose et leur résistance à l'acide acétique, qui sont évalués comme indiqué en (iii) et (iv). Les hybrides sélectionnés sont les suivants :

- les hybrides qui ont un retard d'initiation de la fermentation alcoolique inférieur à 30 heures,
- les hybrides qui convertissent au moins 60 % du xylose à 60 heures.

(vi) Evolution dirigée

L'évolution dirigée est la combinaison d'une étape de mutagenèse aux UV suivie d'un enrichissement sur un milieu de fermentation dont le xylose constitue la seule source de carbone.

Étape 1 : Lors de la mutagenèse aux UV, 15 mL d'une suspension de cellules de levure à 5 % (en g de matière sèche pour 100g de la suspension) sont placés dans une boite de pétri de 9 cm. La boîte de pétri est ensuite placée sous un rayonnement UV de 300 J / cm2 à 254 nm.

Étape 2 : Les cellules de levure irradiées sont ensuite inoculées à hauteur de 1 g de matière sèche / kg de milieu YFX. Après 96 heures de multiplication en condition d'hypoxie à 32°C, 100 $\mu$L du milieu de culture sont utilisés pour inoculer 50 mL d'un milieu glycérol comprenant 20g/kg de glycérol, 5g/kg de sulfate d'ammonium et 1,7 g/kg de milieu YNB (*Yeast Nitrogen Base*) de Difco. Cette seconde culture est réalisée en condition aérobie et à 30°C pendant 48 heures.

1 ml de cette culture est ensuite prélevé pour inoculer 170 ml de milieu YFX.

Ce cycle est répété 8 fois.

**Résultats**

Plusieurs hybrides sont obtenus par croisement de la souche 1-4538 avec la souche 1-4627, parmi lesquels les hybrides H1 et H2.

L'hybride H2 qui n'est pas apte à métaboliser le xylose au sens de l'invention mais présente une résistance à l'acide acétique est soumis à une étape d'évolution dirigée : les hybrides ED1, ED2 et ED3 sont alors obtenus, correspondant respectivement aux souches de levure I-4624, I-4626 et 1-4625.

Le tableau 1 indique le pourcentage de xylose converti en éthanol à 60 heures dans le milieu YFGX et le retard d'initiation de la fermentation alcoolique dans le milieu YFAc (en heures) de différentes souches de levure :

- le témoin Ac qui est une souche de levure non génétiquement modifiée résistante à l'acide acétique et qui n'est donc pas capable de métaboliser le xylose,
- la souche de levure de départ 1-4538 qui est très performante pour la production d'alcool, mais n'est pas résistante à l'acide acétique,
- la souche de levure de départ 1-4627 qui est résistante à l'acide acétique, mais n'est pas apte à métaboliser le xylose au sens de l'invention,
- l'hybride H1 qui est une souche de levure apte à métaboliser le xylose et qui est résistante à l'acide acétique,
- l'hybride H2 qui n'est pas apte à métaboliser le xylose au sens de l'invention, mais présente une résistance à l'acide acétique,
- les hybrides ED1, ED2 et ED3 qui sont aptes à métaboliser le xylose et sont résistants à l'acide acétique.

Tableau 1

| Souches de levure | Pourcentage de xylose converti en éthanol à 60 heures dans le milieu YFGX | Retard d'initiation de la fermentation alcoolique dans le milieu YFAc (en heures) |
|---|---|---|
| Témoin Ac | 0 | 12 |
| 1-4538 | 95 | 40 |
| 1-4627 | 60 | 18 |
| H1 | 95 | 20 |
| H2 | 60 | 25 |
| ED1 (I-4624) | 95 | 22 |
| ED2 (1-4626) | 95 | 26 |
| ED3 (1-4625) | 95 | 22 |

Par ailleurs, la production d'éthanol des souches de levure est évaluée dans le milieu de fermentation YFGX-Ac comprenant à la fois du glucose, du xylose et de l'acide acétique en tant qu'inhibiteur de fermentation.

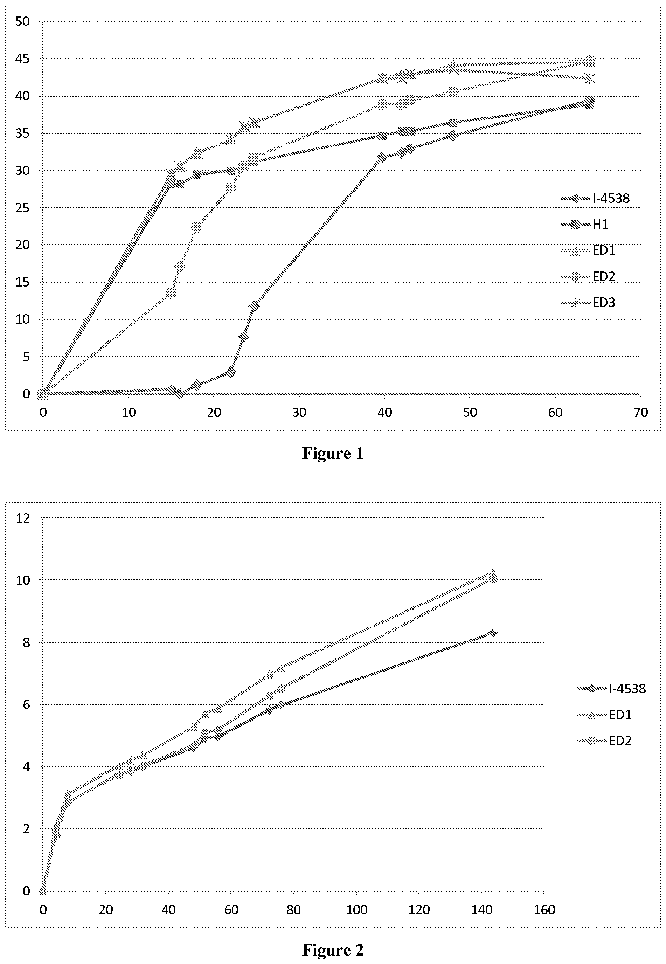

Les souches de levure H1, ED1, ED2 et ED3 ont une production d'alcool nettement améliorée par rapport à la souche de levure parent 1-4538 dans ce milieu YFGX-Ac (*cf. figure 1*).

EXEMPLE 2: PRODUCTION D'ALCOOL EN MILIEU D'APPLICATION REEL A PARTIR DES SOUCHES DE LEVURE SELON L'INVENTION

**Matériel et méthodes**

La production d'alcool des souches de levure ED1, ED2 et ED3 est évaluée en milieu d'application réel.

La production d'alcool de la souche de levure parent 1-4538 est également évaluée à titre comparatif.

L'évaluation de la production d'alcool des souches de levure est réalisée sur deux milieux d'application différents :

- le milieu A comprenant :

  ◦ un substrat hémicellulosique qui correspond à la phase liquide obtenue après séparation solide/liquide de la biomasse ayant subi un prétraitement et une hydrolyse thermochimique d'une paille de blé et qui comprend majoritairement du xylose (48 g/kg), d'autres sucres en faible quantité, ainsi que des inhibiteurs de fermentation issus du prétraitement physico-chimique de la biomasse lignocellulosique, et
  ◦ des nutriments (sources d'azote et phosphore),

- le milieu B correspondant au milieu A ci-dessus supplémenté en glucose, afin de mimer un hydrolysat de biomasse lignocellulosique dont la fraction hémicellulosique a été hydrolysée et dont la fraction cellulosique aurait été partiellement hydrolysée.

Le pH initial des milieux A et B est ajusté à pH 5,5.

Le fermenteur est inoculé avec 1,5 g/kg de matière sèche de la souche de levure à évaluer.

La fermentation alcoolique est conduite à 35°C.

Une fermentation est également conduite à 32°C avec le milieu A.

**Résultats**

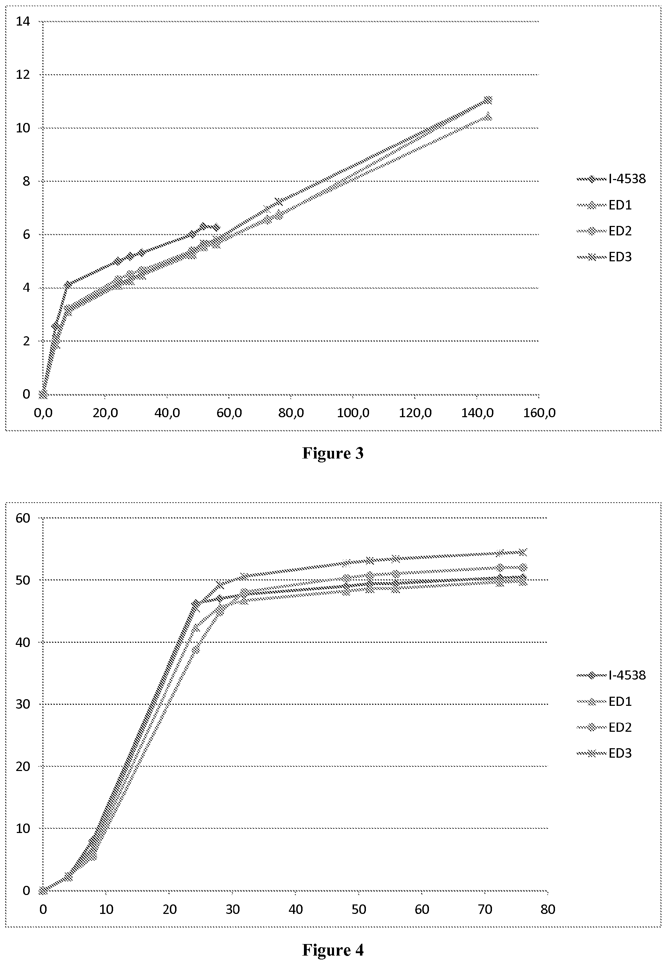

Les résultats obtenus avec le milieu A à 32°C et 35°C sont donnés respectivement dans les figures 2 et 3, et les résultats avec le milieu B à 35°C dans la figure 4.

A noter que dans la figure 3, les points sont arrêtés à 60h pour la souche de levure 1-4538, car la phase de plateau est déjà atteinte.

Les hybrides ED1 et ED2 montrent un réel avantage dans le milieu d'application A à 32°C par rapport à la souche de levure parent 1-4538.

Les hybrides ED1, ED2 et ED3 montrent un réel avantage dans le milieu d'application A à 35°C par rapport à la souche de levure parent 1-4538. En effet, même si la production d'éthanol est meilleure dans les 60 premières heures pour la souche de levure 1-4538, la quantité d'éthanol produit maximale est de 6,3 g par kg de milieu, alors que des quantités d'éthanol supérieures à 10 g/kg sont obtenues avec les hybrides ED1, ED2 et ED3.

Les hybrides ED1 et ED2 montrent à nouveau un réel avantage dans le milieu d'application B à 35°C par rapport à la souche de levure 1-4538. L'hybride ED3 est un peu en retrait dans ce milieu d'application.

**Revendications**

1. Souche de levure déposée le 24 mai 2012 à la CNCM sous le numéro 1-4627.

2. Procédé d'obtention d'une souche de levure apte à métaboliser le xylose et résistante à l'acide acétique, comprenant les étapes de :

   - croisement de la souche de levure déposée le 5 octobre 2011 à la CNCM sous le numéro 1-4538 avec la souche de levure déposée le 24 mai 2012 à la CNCM sous le numéro 1-4627, pour obtenir au moins un hybride,
   - sélection d'au moins un hybride apte à métaboliser le xylose et résistant à l'acide acétique.

3. Procédé selon la revendication 2, **caractérisée en ce que** ladite étape de croisement comprend :

   - une étape de sporulation de la souche de levure déposée le 5 octobre 2011 à la CNCM sous le numéro 1-4538, pour obtenir au moins un ségrégeant X,
   - une étape de sporulation de la souche de levure déposée le 24 mai 2012 à la CNCM sous le numéro 1-4627 pour obtenir au moins un ségrégeant Y,
   - une étape d'hybridation d'au moins un ségrégeant X avec au moins un ségrégeant Y, ledit ségrégeant X étant capable de convertir du xylose en éthanol et/ou ledit ledit ségrégeant Y possédant une résistance à l'acide acétique, pour obtenir au moins un hybride.

4. Procédé selon la revendication 2 ou la revendication 3, **caractérisée en ce que** ladite étape de croisement comprend :

   - une étape de sporulation de la souche de levure déposée le 5 octobre 2011 à la CNCM sous le numéro 1-4538, pour obtenir au moins un ségrégeant X,
   - une étape de sporulation de la souche de levure déposée le 24 mai 2012 à la CNCM sous le numéro 1-4627 pour obtenir au moins un ségrégeant Y,
   - une étape d'hybridation d'au moins un ségrégeant X avec au moins un ségrégeant Y, ledit ségrégeant X convertissant au moins 60% du xylose en éthanol en 60 heures et/ou ledit ségrégeant Y ayant un retard d'initiation de la fermentation alcoolique inférieur à 30 heures, pour obtenir au moins un hybride.

5. Procédé selon l'une quelconque des revendications 2 à 4, **caractérisée en ce que** ladite étape de sélection d'au moins un hybride apte à métaboliser le xylose et résistant à l'acide acétique comprend les étapes de :

   - mesure du pourcentage de xylose converti en éthanol par au moins un hybride en condition anaérobie en 60 heures dans un milieu de fermentation comprenant 55 g de gluclose et 45 g de xylose par kg dudit milieu,
   - mesure du retard d'initiation de la fermentation alcoolique d'au moins un hybride dans un milieu de fermentation comprenant 4000 ppm d'acide acétique à pH 4,4,
   - sélection d'au moins un hybride qui convertit au moins 70% du xylose en éthanol en 60 heures et dont le retard d'initiation de la fermentation alcoolique est inférieur à 30 heures.

6. Procédé selon l'une quelconque des revendications 2 à 5, comprenant en outre une étape d'évolution dirigée.

7. Souche de levure choisie parmi la souche de levure déposée le 24 mai 2012 à la CNCM sous le numéro 1-4624, la souche de levure déposée le 24 mai 2012 à la CNCM sous le numéro 1-4625 et la souche de levure déposée le 24 mai 2012 à la CNCM sous le numéro 1-4626.

8. Procédé de production d'au moins un produit de fermentation comprenant une étape de fermentation en condition anaérobie par une souche de levure selon la revendication 7 dans un milieu de fermentation.

9. Procédé selon la revendication 8, **caractérisé en ce que** ledit milieu de fermentation comprend du xylose et/ou au moins un inhibiteur de fermentation.

10. Procédé selon la revendication 8 ou la revendication 9, **caractérisé en ce que** ledit milieu de fermentation comprend au moins un hydrolysat de tout ou partie d'un matériel végétal.

11. Utilisation d'une souche de levure selon la revendication 7 pour la production d'un produit de fermentation, de préférence dans un milieu de fermentation comprenant du xylose et/ou au moins un inhibiteur de fermentation.

12. Une culture comprenant une souche de levure selon la revendication 7.

13. Un pain de levure comprenant une souche de levure selon la revendication 7.

14. Culture selon la revendication 12, **caractérisée en ce qu'**elle est sous forme de levure sèche.


**Patentansprüche**

1. Am 24. Mai 2012 bei der CNCM unter der Nummer I-4627 hinterlegter Hefestamm.

2. Verfahren zum Erhalt eines Hefestamms, der Xylose verstoffwechseln kann und gegen Essigsäure resistent ist, welches die folgenden Schritte umfasst:

   - Kreuzen des am 5. Oktober 2011 bei der CNCM unter der Nummer I-4538 hinterlegten Hefestamms mit dem am 24. Mai 2012 bei der CNCM unter der Nummer I-4627 hinterlegten Hefestamm zum Erhalt wenigstens eines Hybrids,
   - Selektion wenigstens eines Hybrids, der Xylose verstoffwechseln kann und gegen Essigsäure resistent ist.

3. Verfahren nach Anspruch 2, **dadurch gekennzeichnet, dass** der Schritt der Kreuzung umfasst:

   - einen Schritt der Sporenbildung des am 5. Oktober 2011 bei der CNCM unter der Nummer I-4538 hinterlegten Hefestamms zum Erhalt wenigstens eines X-Segreganten,
   - einen Schritt der Sporenbildung des am 24. Mai 2012 bei der CNCM unter der Nummer I-4627 hinterlegten Hefestamms zum Erhalt wenigstens eines Y-Segreganten,
   - einen Schritt der Hybridisierung wenigstens eines X-Segreganten mit wenigstens einem Y-Segreganten zum Erhalt wenigstens eines Hybrids, wobei der X-Segregant in der Lage ist, Xylose in Ethanol umzuwandeln und/oder wobei der Y-Segregant eine Resistenz gegenüber Essigsäure aufweist.

4. Verfahren nach Anspruch 2 oder Anspruch 3, **dadurch gekennzeichnet, dass** der Schritt der Kreuzung umfasst:

   - einen Schritt der Sporenbildung des am 5. Oktober 2011 bei der CNCM unter der Nummer I-4538 hinterlegten Hefestamms zum Erhalt wenigstens eines X-Segreganten,
   - einen Schritt der Sporenbildung des am 24. Mai 2012 bei der CNCM unter der Nummer I-4627 hinterlegten Hefestamms zum Erhalt wenigstens eines Y-Segreganten,
   - einen Schritt der Hybridisierung wenigstens eines X-Segreganten mit wenigstens einem Y-Segreganten zum Erhalt wenigstens eines Hybrids, wobei der X-Segregant in 60 Stunden wenigstens 60% der Xylose in Ethanol umwandelt und/oder wobei der Y-Segregant eine Verzögerung der alkoholischen Fermentation von weniger als 30 Stunden aufweist, um wenigstens einen Hybrid zu erhalten.

5. Verfahren nach einem der Ansprüche 2 bis 4, **dadurch gekennzeichnet, dass** der Schritt der Selektion wenigstens eines Hybrides, der Xylose verstoffwechseln kann und gegen Essigsäure resistent ist, die folgenden Schritte umfasst:

   - Messen des Prozentsatzes der in Ethanol umgewandelten Xylose durch wenigstens ein Hybrid unter anaeroben Bedingungen in 60 Stunden in einem Fermentationsmedium, welches 55g Glucose und 45g Xylose pro kg des Mediums aufweist,
   - Messen der Verzögerung des Beginns der alkoholischen Fermentation wenigstens eines Hybrides in einem Fermentationsmedium, welches 4000 ppm Essigsäure mit einem pH-Wert von 4,4 umfasst,
   - Selektion wenigstens eines Hybrides, der in 60 Stunden wenigstens 60% der Xylose in Ethanol umwandelt und dessen Verzögerung des Beginns der alkoholischen Fermentation weniger als 30 Stunden beträgt.

6. Verfahren nach einem der Ansprüche 2 bis 5, welches ferner einen Schritt der gesteuerten Entwicklung umfasst.

7. Hefestamm, welcher gewählt ist aus dem am 24. Mai 2012 bei der CNCM unter der Nummer I-4624 hinterlegten Hefestamm, dem am 24. Mai 2012 bei der CNCM unter der Nummer I-4625 hinterlegten Hefestamm und dem am 24. Mai 2012 bei der CNCM unter der Nummer I-4626 hinterlegten Hefestamm.

8. Verfahren zur Herstellung wenigstens eines Fermentationsproduktes, welches einen Schritt der Fermentation durch einen Hefestamm nach Anspruch 7 unter anaeroben Bedingungen in einem Fermentationsmedium umfasst.

9. Verfahren nach Anspruch 8, **dadurch gekennzeichnet, dass** das Fermentationsmedium Xylose und/oder wenigstens einen Fermentationshemmer umfasst.

10. Verfahren nach Anspruch 8 oder Anspruch 9, **dadurch gekennzeichnet, dass** das Fermentationsmedium wenigstens ein teilweise oder ganz aus pflanzlichem Material bestehendes Hydrolysat umfasst.

11. Verwendung eines Hefestamms nach Anspruch 7 für die Herstellung eines Fermentationsproduktes, vorzugsweise in einem Fermentationsmedium, welches Xylose und/oder wenigstens einen Fermentationshemmer aufweist.

12. Kultur, welche einen Hefestamm nach Anspruch 7 aufweist.

13. Hefebrot, welches einen Hefestamm nach Anspruch 7 aufweist.

14. Kultur nach Anspruch 12, **dadurch gekennzeichnet, dass** sie als Trockenhefe vorliegt.


**Claims**

1. Yeast strain deposited on 24 May 2012 at the CNCM] under number 1-4627.

2. Method for obtaining a yeast strain capable of metabolizing xylose and resistant to acetic acid, comprising the steps of:

   - crossing the yeast strain deposited on 5 October 2011 at the CNCM under number 1-4538 with the yeast strain deposited on 24 May 2012 at the CNCM under number 1-4627, so as to obtain at least one hybrid,
   - selecting at least one hybrid capable of metabolizing xylose and resistant to acetic acid.

3. Method according to Claim 2, **characterized in that** said crossing step comprises:

   - a step of sporulation of the yeast strain deposited on 5 October 2011 at the CNCM under number 1-4538, so as to obtain at least one segregant X,
   - a step of sporulation of the yeast strain deposited on 24 May 2012 at the CNCM under number 1-4627, so as to obtain at least one segregant Y,
   - a step of hybridization of at least one segregant X with at least one segregant Y, said segregant X being capable of converting xylose to ethanol and/or said segregant Y having resistance to acetic acid, so as to obtain at least one hybrid.

4. Method according to Claim 2 or Claim 3, **characterized in that** said crossing step comprises:

   - a step of sporulation of the yeast strain deposited on 5 October 2011 at the CNCM under number 1-4538, so as to obtain at least one segregant X,
   - a step of sporulation of the yeast strain deposited on 24 May 2012 at the CNCM under number 1-4627, so as to obtain at least one segregant Y,
   - a step of hybridization of at least one segregant X with at least one segregant Y, said segregant X converting at least 60% of xylose to ethanol in 60 hours and/or said segregant Y having a delay of initiation of alcoholic fermentation of less than 30 hours, so as to obtain at least one hybrid.

5. Method according to any one of Claims 2 to 4, **characterized in that** said step of selecting at least one hybrid capable of metabolizing xylose and resistant to acetic acid comprises the steps of:

- measuring the percentage of xylose converted to ethanol by at least one hybrid under anaerobic conditions in 60 hours in a fermentation medium comprising 55 g of glucose and 45 g of xylose per kg of said medium,
- measuring the delay of initiation of alcoholic fermentation of at least one hybrid in a fermentation medium comprising 4000 ppm of acetic acid at pH 4.4,
- selecting at least one hybrid which converts at least 70% of xylose to ethanol in 60 hours and of which the delay of initiation of alcoholic fermentation is less than 30 hours.

6. Method according to any one of Claims 2 to 5, further comprising a directed evolution step.

7. Yeast strain chosen from the yeast strain deposited on 24 May 2012 at the CNCM under number 1-4624, the yeast strain deposited on 24 May 2012 at the CNCM under number 1-4625 and the yeast strain deposited on 24 May 2012 at the CNCM under number 1-4626.

8. Method for producing at least one fermentation product, comprising a step of fermentation under anaerobic conditions by a yeast strain according to Claim 7 in a fermentation medium.

9. Method according to Claim 8, **characterized in that** said fermentation medium comprises xylose and/or at least one fermentation inhibitor.

10. Method according to Claim 8 or Claim 9, **characterized in that** said fermentation medium comprises at least one hydrolyzate of all or part of a plant material.

11. Use of a yeast strain according to Claim 7, for producing a fermentation product, preferably in a fermentation medium comprising xylose and/or at least one fermentation inhibitor.

12. Culture comprising a yeast strain according to Claim 7.

13. Block yeast comprising a yeast strain according to Claim 7.

14. Culture according to Claim 12, **characterized in that** it is in the form of dry yeast.

**Figure 1**

**Figure 2**

Figure 3

Figure 4

**EP 2 855 711 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2011071616 W

- DE 102008031350

**Littérature non-brevet citée dans la description**

- **ALMEIDA ; HAHN-HÄGERDAL.** *International Sugar Journal,* 2009, vol. 111 (1323
- **TOYOMA et al.** *Bioresour. Technol.,* 2011, vol. 12, 7917-7924

- Sporulation and Hydridization of Yeast. **R.R. FOWELL.** The Yeasts. Academic Press, 1969, vol. 1
- **G. REED ; T.W. NAGODAWITHANA.** Yeast Technology. Van Nostrand Reinhold, 1991